# EUROPÄISCHE PATENTSCHRIFT

(11) **EP 0 520 414 B1**
(45) Veröffentlichungstag und Bekanntmachung des Hinweises auf die Patenterteilung: **13.03.1996**
(21) Anmeldenummer: 92110652.2
(22) Anmeldetag: 24.06.1992
(51) Int. Cl.: C07C 69/95, A61K 31/235

(54) **Verfahren zur Gewinnung von Diacetylrhein**
Process for the preparation of diacetylrhein
Procédé d'obtention de diacétylrhéine

(30) Priorität: 25.06.1991 DE 4120989
(43) Veröffentlichungstag der Anmeldung: 30.12.1992
(73) Patentinhaber: MADAUS Aktiengesellschaft, D-51075 Köln (DE)
(72) Erfinder: Carcasona, Alfons, Dr., W-5000 Köln 80 (DE); Grimminger, Wolf, Dr., W-5060 Bergisch-Gladbach 1 (DE); Hietala, Pentti, Dr., SF-00660 Helsnki 66 (FI); Witthohn, Klaus, Dr., W-5063 Overath (DE); Zaeske, Helga, W-5063 Overath 3 (DE)

(56) Entgegenhaltungen:
- FR-A- 2 508 798
- GB-A- 2 112 640
- TETRAHEDRON, Bd. 23, Nr. 1, Januar 1967, OXFORD GB Seiten 515 - 518; V.K.MURTY ET AL: 'Chemical examination of Cassia fistula'

## Beschreibung

Die Erfindung betrifft ein Verfahren zur Gewinnung von Diacetylrhein in pharmazeutisch brauchbarer Reinheit mit einem Restgehalt an unerwünschten Aloeemodinderivaten von insgesamt weniger als 20 ppm, das nach diesem Verfahren erhältliche Diacetylrhein und ein pharmazeutisches Mittel, das diese Verbindung enthält.

Diacetylrhein der Formel: 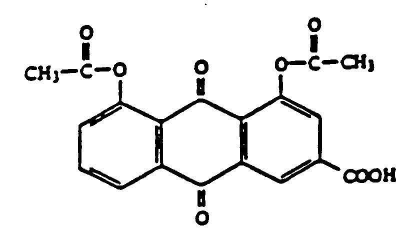
ist ein Arzneiwirkstoff, der antiarthritische, antiinflammatorische, antipyretische und analgetische Aktivität besitzt. Diacetylrhein wird daher zur Behandlung von Arthritiserkrankungen eingesetzt, siehe beispielsweise DE-A 27 11 493 und US-A- 4 244 968.

Diacetylrhein läßt sich beispielsweise durch Acetylierung von Barbaloin und Oxidation des erhaltenen peracetylierten Barbaloins mit Chromtrioxid herstellen. Außerdem läßt sich Diacetylrhein durch Acetylierung von Rhein herstellen, das beispielsweise aus der Sennadroge gewonnen werden kann.

In dem nach diesem Verfahren erhaltenen Diacetylrhein sind als unerwünschte Begleitstoffe Aloeemodinderivate enthalten, die von einer unvollständigen Oxidation mit Chromtrioxid herrühren oder bei der Extraktion der Sennadroge mitextrahiert werden. Diese Begleitstoffe sind in relativ geringen Mengen enthalten und lassen sich daher anhand klassischer Reinigungsoperation nur sehr schwierig abtrennen. Außerdem fallen bei dem oben zuerst genannten Verfahren Chromrückstände an, die in geeigneter Weise entsorgt werden müssen.

Der vorliegenden Erfindung liegt daher die Aufgabe zugrunde, ein Verfahren zur Gewinnung von Diacetylrhein zur Verfügung zu stellen, das einfach und mit hoher Ausbeute durchzuführen ist und bei dem das Diacetylrhein in pharmazeutisch brauchbarer Reinheit mit einem Restgehalt an unerwünschten Aloeemodinderivaten von insgesamt weniger als 20 ppm anfällt.

Diese Aufgabe wird gelöst durch das erfindungsgemäße Verfahren, das dadurch gekennzeichnet ist, daß man Aloeemodinkomponenten (d.h. Aloeemodin und/oder Derivate davon) enthaltendes Diacetylrhein einer Flüssig-Flüssig-Verteilung in einem mit Wasser nur teilweise mischbaren polaren organischen Lösungsmittel und einer wäßrigen Phase von pH 6,5 bis 7,5 unterwirft und das Diacetylrhein gewinnt und gegebenenfalls umkristallisiert.

Ein Aloeemodin enthaltendes Diacetylrhein kann beim erfindungsgemäßen Verfahren eingesetzt werden. Eine wesentliche Quelle für Diacetylrhein sind die in der Sennadroge enthaltenen Sennoside sowie das aus den Sennosiden erhältliche Rhein-9-anthron-8-glucosid.

Eine bevorzugte Ausführungsform der Erfindung ist daher ein Verfahren zur Herstellung von Diacetylrhein, das im wesentlichen frei von Aloeemodinderivaten ist, wobei man
A) Aloeemodinkomponenten enthaltendes Rhein-9-anthron-8-glucosid zu den entsprechenden Anthrachinonverbindungen oxidiert,
B) den Glucoserest in 8-Stellung der Anthrachinonverbindungen in saurem Medium abspaltet,
C) die erhaltenen 1,8-Dihydroxyanthrachinonverbindungen acetyliert und
D) eine Flüssig-Flüssig-Verteilung des erhaltenen Produktes zwischen einem nur teilweise mit Wasser mischbaren, polaren organischen Lösungsmittel und einer wäßrigen Phase von pH 6,5 bis 7,5 vornimmt und das Diacetylrhein gewinnt und gegebenenfalls umkristallisiert.

Eine weitere bevorzugte Ausführungsform der Erfindung ist ein Verfahren zur Herstellung von Diacetylrhein, das im wesentlichen frei von Aloeemodinderivaten ist, wobei man
A) ein Sennosidgemisch einer Reduktion zu den entsprechenden Anthronverbindungen unterwirft,
B) die erhaltenen Anthronverbindungen zu den entsprechenden Anthrachinonverbindungen oxidiert,
C) den Glucoserest in 8-Stellung der Anthrachinonverbindungen in saurem Medium abspaltet,
D) die erhaltenen 1,8-Dihydroxyanthrachinonverbindungen acetyliert und
E) eine Flüssig-Flüssig-Verteilung des erhaltenen Produktes zwischen einem nur teilweise mit Wasser mischbaren, polaren organischen Lösungsmittel und einer wäßrigen Phase von pH 6,5 bis 7,5 vornimmt und das Diacetylrhein gewinnt und gegebenenfalls umkristallisiert.

Im folgenden werden die einzelnen Stufen des erfindungsgemäßen Verfahrens erläutert:

### Reduktion des Sennosidgemisches zu den entsprechenden Anthronverbindungen

Das als Ausgangsmaterial dienende Sennosidgemisch läßt sich beispielsweise aus der Sennadroge gewinnen.

Die Sennadroge besteht aus den getrockneten Blättern und Früchten der Sennespflanze, beispielsweise der indischen Senna (Cassia angustifolia) und der ägyptischen Senna (Cassia acutifolia). Die Sennadroge enthält Dianthronglucoside von Rhein und Aloe-Emodin. Die wichtigsten sind die Sennoside A, B, A1, C, D und D1. Die Sennoside entsprechen der Formel: 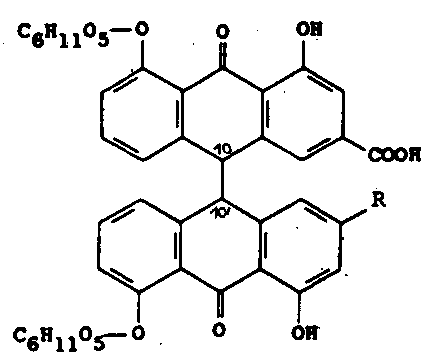
Bei den Sennosiden A, B und A1 steht R für COOH und bei den Sennosiden C, D und D1 steht R für CH₂OH. Die Sennoside A, B und A1 bzw. C, D und D1 sind Stereoisomere und unterscheiden sich untereinander durch die Konfiguration an den C-Atomen 10 und 10'.

Die Gewinnung der Sennoside aus der Sennadroge ist beispielsweise in der DE-A-32 00 131 beschrieben, auf die hiermit in vollem Umfang Bezug genommen wird. Danach extrahiert man zunächst die Sennadroge mit wäßrigem Methanol. Das nach vollständigem Entfernen des Methanols verbleibende Konzentrat enthält die Sennoside in Form eines Alkalimetallsalzes vorzugsweise Kaliumsalz. Das Konzentrat wird durch Flüssig-Flüssig-Extraktion mit in Wasser teilweise löslichen Alkoholen oder Ketonen (z.B. Butanol-2, 2-Butanon) gereinigt (Raffinat). Das Raffinat wird auf einen pH von etwa 1,5 bis 2,0 angesäuert und die Sennoside werden unter Animpfen zur Kristallisation gebracht. Das erhaltene Rohsennosidgemisch ist als Ausgangsprodukt für das erfindungsgemäße Verfahren brauchbar. Gewünschtenfalls kann man das Rohsennosidgemisch auch noch umkristallisieren.

Alternativ kann man das mit einem in Wasser teilweise löslichen Alkohol oder Keton, insbesondere Butanol-2, versetzte Konzentrat als Ausgangsprodukt für das erfindungsgemäße Verfahren verwenden.

Bei der Extraktion der Sennadroge beträgt das Verhältnis von Droge zu Extraktlösungsmittel vorzugsweise 1 : 4 bis 1 : 15, insbesondere 1 : 4 bis 1 : 10.

Die Extraktion wird vorzugsweise in Gegenwart eines Puffers durchgeführt, beispielsweise Trinatriumcitrat, Glycin, Natriumbikarbonat oder Saccharose.

Nach den erfindungsgemäßen Verfahren werden diese Ausgangsmaterialien einer Reduktion zu Rhein-9-anthron-8-glucosid (R=COOH) und Aloeemodin-9-anthron-8-glucosid (R=CH₂OH) der Formel: 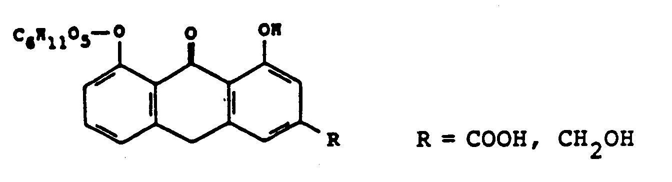
unterworfen.

Reduktionsmittel mit einem geeigneten Reduktionspotential sind Zinn-II-chlorid, Schwefeldioxid, Alkalimetallborwasserstoffe und vorzugsweise Alkalimetalldithionite, insbesondere Natriumdithionit. Das Reduktionsmittel kommt in großem Überschuß zur Anwendung. Dithionite, insbesondere Natriumdithionit, verwendet man im allgemeinen in der 1- bis 4-fachen Gew.-Menge, bezogen auf den Gehalt des Ausgangsmaterials an Sennosiden.

Zur Durchführung der Reduktion kann man das Ausgangsmaterial in wäßriger Lösung oder Suspension vorlegen und das Reduktionsmittel in fester Form oder in Wasser gelöst zugeben. Vorzugsweise wird in einem 2-Phasen-Gemisch gearbeitet, indem man ein mit Wasser höchstens teilweise mischbares, polares organisches Lösungsmittel, insbesondere 2-Butanol, zugibt.

Die Reduktion wird insbesondere bei 40 - 60 °C, insbesondere bei 50 - 55 °C und bei einem pH von 7 bis 9 durchgeführt. Vorzugsweise führt man die Reduktion mehrfach, insbesondere 2 bis 10 mal durch.

Die gebildeten 9-Anthron-8-glucoside fällt man durch Zugabe einer Säure, beispielsweise Schwefelsäure, bis pH 4 bis 4,5 aus. Die Temperatur sollte dabei zweckmäßigerweise nicht mehr als 40 °C betragen. Zweckmäßigerweise arbeitet man bei der Ausfällung der Anthronglucoside und bei ihrer Isolierung (beispielsweise durch Filtration) unter Stickstoff, um eine unkontrollierte Oxidation dieser Verbindung zu vermeiden.

### Oxidation der Anthronverbindungen zu den Anthrachinonverbindungen

Die erhaltenen Anthronverbindungen werden nun zu den entsprechenden Anthrachinonverbindungen der Formel: 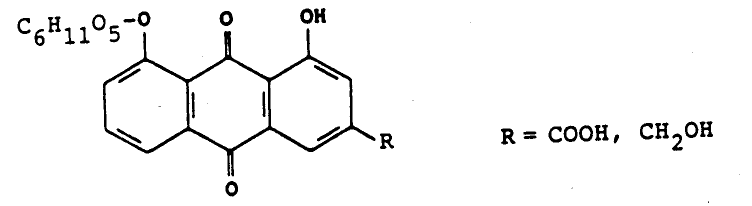
oxidiert. Geeignete Oxidationsmittel für diesen Zweck sind z.B. Sauerstoff, Peroxidverbindungen (Wasserstoffperoxid), Mangan-, Chrom-oder Eisenverbindungen in den hohen Oxidationsstufen. Vorzugsweise verwendet man ein Eisen-III-Salz, insbesondere Eisen-III-Sulfat. Zweckmäßigerweise arbeitet man bei erhöhter Temperatur, jedoch unterhalt 60°C. Dadurch wird vermieden, daß unerwünschte und undefinierbare Oxidationsprodukte entstehen. Nach beendeter Oxidation werden die gebildeten Anthrachinon-8-glucoside in üblicher Weise isoliert.

### Abspaltung des Glucoserestes

Der Glucoserest in 8-Position der Anthrachinonverbindungen wird in saurer Lösung abgespalten. Zweckmäßigerweise arbeitet man bei etwa 85 bis 95 °C. Das erhaltene Produkt wird in üblicher Weise isoliert.

Es ist bekannt, Sennoside nach saurer Hydrolyse durch Umsetzung mit Eisen-III-Chlorid direkt in Rhein zu überführen, siehe beispielsweise DE-A-27 11 493. Dabei beträgt die Ausbeute jedoch nur etwa 10 % und außerdem ist das gebildete Rhein schwer abzutrennen.

Beim erfindungsgemäßen Verfahren wird die reduktive Spaltung der Sennoside, die Oxidation der gebildeten Anthronverbindungen zu den entsprechenden Anthrachinon-Verbindungen und die Abspaltung des Glucoserestes in 8-Stellung der Anthrachinonverbindungen in jeweils getrennten Stufen durchgeführt. Überraschenderweise erhält man auf diese Weise Rhein in einer Ausbeute von 89 %. Zudem ist es möglich, die Oxidation bei schonenden Temperaturen durchzuführen, so daß die Bildung von unerwünschten und undefinierbaren Oxidationsprodukten vermieden wird. Außerdem kann bei dieser Reaktionsführung das eingesetzte Eisensalz nahezu quantitativ wiedergewonnen und nach Rückoxidation erneut verwendet werden. Die Trennung von Oxidationsschritt und Hydrolyseschritt erlaubt es, aufgrund der höheren Wasserlöslichkeit der Anthronglucoside im Vergleich zu den betreffenden Aglyka, die Oxidation schonend bei Raumtemperatur unter 60°C durchzuführen, wodurch die sonst unvermeidliche Bildung undefinierbarer Nebenprodukte vermieden wird.

### Acetylierung der 1,8-Dihydroxyanthrachinonverbindung

Die Acetylierung der erhaltenen 1,8-Dihydroxyanthrachinonverbindungen erfolgt in üblicher Weise. Beispielsweise kann man mit Acetanhydrid in Gegenwart von Natriumacetat acetylieren, wie in Arch. Pharm. 241, 607 (1903) beschrieben. Die Acetylierung kann jedoch auch mit anderen, dem Fachmann bekannten Methoden erfolgen, beispielsweise durch Umsetzung mit Acetylchlorid etc.

### Flüssig-Flüssig-Verteilung

Man führt eine flüssig-flüssig-Verteilung des erhaltenen Produktes in einem mit Wasser höchstens teilweise mischbaren polaren organischen Lösungsmittel und einer wäßrigen Phase von pH 6,5 bis 7,5 durch. Geeignete polare organische Lösungsmittel sind C₄-C₅-Alkanole und C₁-C₃-Dialkylketone, wie 1-Butanol, 2-Butanol, Isobutanol und 2-Butanon. Letzteres ist bevorzugt.

Das Volumenverhältnis von schwerer zu leichter Phase liegt im allgemeinen im Bereich von 1:2 bis 2:1. Die leichtere Phase ist eine Lösung der Diacetylanthrachinonverbindungen in dem polaren organischen Lösungsmittel. Als schwerere Phase dient eine wäßrige Phase von pH 6,5 bis 7,5, der vorzugsweise mit einem Puffer, insbesondere einem Acetatpuffer eingestellt wird.

Vorzugsweise erfolgt die flüssig-flüssig-Extraktion im Gegenstrom. Das Diacetylrhein wird dabei in der organischen Phase in einer Konzentration von etwa 0,01 M zugeführt.

Nach der Verteilung findet sich das gewünschte Diacetylrhein in der schwereren Phase. Es wird durch Ansäuern auf etwa pH 5,2 gefällt und in üblicher Weise gewonnen und als Alkalimetallsalz, vorzugsweise Kaliumsalz umkristallisiert, indem das gebildete Salz in die unlösliche freie Säure überführt wird. Als Variante kann man eine direkte Umkristallisation mit Ethyllactat einsetzen.

Das auf diese Weise erhaltene Diacetylrhein ist im wesentlichen frei von Aloeemodin und Derivaten davon. Der Gehalt an diesen Verunreinigungen beträgt dabei noch ca. 50 ppm (bestimmt nach den in den Beispielen beschriebenen Analyseverfahren). Der Gehalt an diesen Verunreinigungen kann weiter gesenkt werden, wenn man das erhaltene Diacetylrhein auf folgende Weise umkristallisiert. Man überführt das Diacetylrhein in ein Alkalimetallsalz, indem man es mit einer geeigneten Base behandelt. Eine geeignete Base ist beispielsweise ein Alkalimetallacetat, vorzugsweisse Kaliumacetat. Man verwendet vorzugsweise äquimolare Mengen der Base und einen wäßrigen C₁-C₃-Alkohol, beispielsweise 80- bis 90%iges Ethanol, als Reaktionsmedium. Man läßt das Alkalimetallsalz des Diacetylrhein in der Kälte auskristallisieren, nimmt es in einem wäßrigen C₁-C₃-Alkohol auf und fällt es durch Zusatz einer Säure bis zu einem pH-Wert von etwa 3. Das ausgefallene Diacetylrhein wird dann in üblicher Weise isoliert und aufgearbeitet. Als Variante kann man eine direkte Umkristallisation mit Ethyllactat einsetzen.

Das so erhaltene Produkt enthält weniger als 20 ppm der oben erwähnten Verunreinigungen. Außerdem liegt das Produkt in Form von nadelförmigen Kristallen vor, die für die galenische Formulierung besonders geeignet sind.

Das Produkt kann in üblicher Weise getrocknet werden. Zweckmäßigerweise wird man zunächst die Trocknung im Vakuum bei relativ niedriger Temperatur, beispielsweise nicht mehr als 40°C so lange durchführen, bis der Wassergehalt des Produktes auf ca. 3 % oder weniger gefallen ist. Anschließend kann man die Temperatur auf 70 bis 110°C erhöhen.

Die Erfindung betrifft auch das erfindungsgemäß erhältliche, im wesentlichen reine Diacetylrhein sowie ein pharmazeutisches Mittel, das diese Verbindung enthält. Die Anwendungsgebiete, die zu verabreichende Dosis und geeignete Dosierungsformen sind bekannt, siehe US-A-4244968, 4346103, 4950687, DE-A-2711493 sowie Drugs Exptl. Clin. Res. 6 (1) 53 bis 64 (1980).

Die nachfolgenden Beispiele erläutern die Erfindung.

### Beispiel 1

Gewinnung des als Ausgangsmaterial verwendeten Sennosidgemisches:
Man gibt jeweils 40 kg Sennadroge (Sennosidgehalt ca. 1,5%) in zwei in Reihe geschaltete Perkolatoren mit einem Volumen von 250 l und bedeckt sie mit einer gelochten Stahlplatte. Als Lösungsmittel für die Extraktion verwendet man 70%iges Methanol, das auf die Droge im ersten Perkolator geleitet wird. Die im ersten Perkolator gebildete Lösung leitet man auf die Droge, die sich im zweiten Perkolator befindet. Dabei läßt man das Lösungsmittel frei durch den ersten Perkolator fließen.

Für die Extraktion von 40 kg Sennadroge verwendet man insgesamt 160 l Lösungsmittel. Nachdem man dieses Volumen 70%iges Methanol durch die beiden Perkolatoren geleitet und die entsprechende Menge Perkolat aufgefangen hat, koppelt man den Entleerungsschlauch des Perkolators mit einem Nachperkolatbehälter und leitet noch zusätzlich 60 l 70%iges Methanol durch die Perkolatoren. Danach leitet man das restliche freie Lösungsmittel aus dem ersten Perkolator in den oberen Teil des zweiten Perkolators und sammelt das Nachperkolat, bis es insgesamt 120 l ausmacht. Dann entleert man den ersten Perkolator, füllt ihn erneut mit 40 kg Sennadroge und pumpt das Nachperkolat auf die Droge, wobei 120 l Nachperkolat ausreichen, um die Droge im Perkolator zu bedecken. Anschließend bringt man die Temperatur der Lösung auf +30°C.

Man verbindet diesen Perkolator mit dem zuvor extrahierten und führt die Extraktion wie oben beschrieben aus.

Für jeweils 40 kg Droge sammelt man 150 l Perkolat, aus dem man das Methanol in einem Vakuumrotationsverdampfer, der mit einer Füllkörpersäule ausgestattet ist, entfernt. Man erhält ca. 30 l Bodenprodukt (Konzentrat), das man in einem "Mixer-Settler"-Apparat (10 Stufen) mit 40 l Butanol-2, das mit Wasser gesättigt ist, extrahiert. Man erhält etwa 38 - 40 l wäßriges Raffinat und etwa 30 - 32 l Butanol-2-Extrakt.

Das wäßrige Raffinat wird mit 93%iger Schwefelsäure unter Rühren während 20 h angesäuert, wobei man 1,6 Vol.-% (bezogen auf das anzusäuernde Flüssigkeitsvolumen) verwendet. Die angesäuerte Lösung hat dann einen pH von 1,5 bis 2,0. Man rührt weitere 6 Tage, läßt den Niederschlag über Nacht absetzen, filtriert ihn, wäscht mit Wasser bis das Waschwasser farblos ist, wäscht mit Methanol und trocknet im Luftstrom bei Zimmertemperatur. Die Ausbeute pro 40 kg Rohmaterial beträgt 760 bis 790 g (Trockensubstanz) Rohsennoside mit einem Sennosidgehalt von 90 bis 94%. Die Ausbeute macht somit etwa 70 % der im Rohmaterial vorhandenen Sennosidmenge aus.

### Stufe A: Reduktion der Sennoside zu Rhein-9-anthron-8-glucosid

9,0 kg Natriumdithionit werden in 100 l entmineralisiertem Wasser gelöst. Dieser Lösung werden unter Rühren die erhaltenen Rohsennoside, enthaltend ca. 3,0 kg Sennoside A, A₁ und B zudosiert. Die homogene Lösung wird 2 Std. bei 55 - 58°C gerührt, dann auf 50 - 55°C gekühlt. Dann wird mit 96 - 98% gew.-prozentiger Schwefelsäure bei pH 4,2 gefällt. Die entstandene Suspension wird weiter 1,5 Std. bei max. 25°C gerührt und dann unter Stickstoff filtriert. Der Rückstand wird mit 50 l entmineralisiertem Wasser, das mit Schwefelsäure auf pH 2 eingestelt worden ist, gewaschen. Anschließend wird mit 10 l Eisen-III-Sulfatlösung (Herstellung siehe Stufe B) überdeckt.

### Stufe B: Oxidation zu Rhein-8-glucosid

Das Produkt der vorausgehenden Stufe wird in einer Lösung aus 184 l entmineralisiertem Wasser und 75,5 kg Eisen-III-Sulfathydrat (22 % Fe³⁺) suspendiert. Die Suspension wird auf 55 - 62°C erhitzt und 14 Std. unter Anwendung eines schnell laufenden Dispergators oxidiert. Ist die Oxidation beendet, wird das gebildete Rhein-8-glucosid abfiltriert und mit 50 l entmineralisiertem Wasser, das mit Schwefelsäure auf pH 2 eingestellt worden ist, gewaschen.

### Stufe C: Hydrolyse zu Rhein

Der feuchte Filterrückstand aus Stufe B wird in 200 kg 20 gew.-prozentiger Schwefelsäure suspendiert und 8 Std. bei 88 - 92°C eingerührt. Das gebildete Rhein wird abfiltriert und kann zur Lagerung bei 1 mbar Vakuum 48 Std. bei 40°C getrocknet oder auch im feuchten Zustand sofort zur Acetylierung in Stufe D eingesetzt werden.

Gesamtausbeute für die Stufen A bis C: 89 %, bezogen auf in Stufe A eingesetzte Sennoside A, A₁ und B.

### Stufe D: Acetylierung zu Diacetylrhein

6,5 kg Rhein aus Stufe C werden in 100 l Essigsäureanhydrid 10 Min. suspendiert, mit 2 kg Kaliumacetat versetzt, auf 95°C unter Rühren erwärmt, mit 0,65 kg Aktivkohle versetzt und 1/2 Std. bei 90 - 95°C gerührt. Die Aktivkohle wird aus der heißen Lösung herausfiltriert und das Filtrat bei 90°C mit 2,1 kg 96 - 98 gew.-prozentiger Schwefelsäure versetzt. Anschließend wird unter Rühren möglichst schnell auf 20°C gekühlt. Die entstandene Suspension wird filtriert. Der Rückstand wird mit entmineralisiertem Wasser sulfatfrei gewaschen.
Ausbeute: 83 %.

### Stufe E: Entfernung von freiem und acetyliertem Aloeemodin

Der Aloeemodinanteil wird durch Gegenstromextraktion auf einer gepulsten Extraktionskolonne mit mindestens 15 theoretischen Böden entfernt. Das Volumenverhältnis von schwerer zu leichter Phase ist 1 : 1. Als schwere Phase dient eine butanon-2-gesättigte 0,1 molare, wäßrige Kaliumacetatlösung. In der leichten Phase, die aus wassergesättigtem Butanon-2 besteht, wird das zu reinigende Diacetylrhein 0,01-molar gelöst. Aus der ablaufenden schweren Phase wird mit 10 gew.-prozentiger Schwefelsäure bei pH 5,2 das Diacetylrhein gefällt. Der Niederschlag wird abfiltriert und mit entmineralisiertem Wasser sulfatfrei gewaschen.

Ausbeute 88 %, bezogen auf eingesetztes Rohdiacetylrhein aus Stufe D.

### Stufe F: Umkristallisation, Trocknung, Vermahlung

### Variante A

Unter schnellem Rühren werden 7,5 kg Diacetylrhein aus Stufe E (bezogen auf getrocknete Substanz) in 250 l 90 vol.-prozentigem Ethanol suspendiert. Die Suspension wird auf 70°C erhitzt und dann mit 3,75 kg Kaliumacetat versetzt. Beim Abkühlen auf 0 - 2°C kristallisiert aus der zwischendurch entstandenen, klaren Lösung das reine Kaliumsalz des Diacetylrheins aus.

Das Kaliumsalz wird abfiltriert und in 300 l 40 vol.-prozentigem Ethanol unter Zusatz von 3 kg Kaliumacetat bei 20 - 30°C gelöst. Die klare Lösung wird mit 10 gew.-prozentiger Schwefelsäure auf pH 3,0 eingestellt. Das auskristallisierte Diacetylrhein wird abfiltriert und mit entmineralisiertem Wasser sulfatfrei gewaschen.

### Variante B:

7,5 kg Diacetylrhein werden in 275 l Ethyllactat suspendiert, durch Erwärmen in Lösung gebracht, filtriert und unter Rühren bis 20 - 25°C kristallisiert. Das auskristallisierte Diacetylrhein wird abfiltriert und mit entmineralisiertem Wasser gewaschen.

Die Trocknung des Produkts erfolgt zunächst im Vakuum bei 1 mbar und 40°C innerhalb 24 Std. Ist der Restwassergehalt unter 3 % gefallen, wird das Material grob zerkleinert und bei 1 mbar Vakuum und 70°C 24 Std. nachgetrocknet.

Anschließend wird bei einer Siebeinlage von 0,5 mm vermahlen und zur Entfernung von Lösungsmittelrückständen bei 1 mbar Vakuum und 70°C nachgetrocknet.
Ausbeute 95 %

### Beispiel 2

Man wiederholt das im Beispiel 1 beschriebene Verfahren, wobei man jedoch folgende Abwandlungen vornimmt:
Bei der Extraktion der Sennadroge verwendet man Trinatriumcitrat, indem man 2,85 kg Trinatriumcitrat vor der Lösungsmittelzugabe zu jeweils 40 kg der Droge gibt. Als Lösungsmittel verwendet man dabei auf 60°C erwärmtes 70%iges Methanol. Nach Entfernen des Methanols bis auf ein Restvolumen von 11,4 l versetzt man das Konzentrat mit ca. 2 l Butanol-2.

Die Reduktion des Sennesfrüchtekonzentrat/Butanol-2-Gemisches wird dann in 7 Stufen unter Stickstoff als Schutzgas durchgeführt. Nach der Reduktionsstufe I erfolgt eine Fällung des rohen Rhein-9-anthron-8-glucosids. Die Reduktionsstufen II bis VII dienen zur teilweisen Entfernung von Aloeemodinderivaten. Diese Stufen werden ohne Fällungen durchgeführt. Die abschließende Fällung des gereinigten Rhein-9-anthron-8-glucosids erfolgt nach der letzten Reduktionsstufe.

### Reduktionsstufe I:

100 l Sennesfrüchtekonzentrat/Butanol-2-Gemisch, enthaltend ca. 4 kg Sennoside, werden in einem Rührbehälter vorgelegt und mit Stickstoff überlagert. Unter Rühren werden 6 l 20%ige (w) Natronlauge, danach 350 l wassergesättigtes Butanol-2 (z.B. aus Stufe II) nacheinander zugesetzt und 15 Min. gerührt. Der Ansatz wird auf 42 - 50°C aufgeheizt und mit 7 kg Natriumdithionit versetzt. Es wird noch 45 Min. gerührt. Der pH-Wert wird mit 20%iger (w) Natronlauge bei 7,5 - 8 gehalten. Das Reduktionspotential (gegen Ag/AgCl-Elektrode) wird erforderlichenfalls durch Natriumdithionitzugabe auf unter - 630 mV gehalten. Nach Kühlung auf 30 - 35°C wird mit 10%iger (w) Schwefelsäure bis pH < 4 innerhalb 1,5 Std. gefällt.

Die entstehende Suspension wird bei kleiner Rührgeschwindigkeit < 25°C ca. 10 Std. gerührt. Der entstandene Niederschlag wird abfiltriert. Der Niederschlag wird in 60 l 15%igem (w) Butanol-2 suspendiert, 30 Min. bei 50 bis 60°C gerührt und anschließend filtriert. Der Rückstand wird mit 100 l demineralisiertem Wasser gewaschen. Die Rohausbeute an Rhein-9-anthron-8-glucosid, bezogen auf eingesetzte Sennoside, liegt über 82 %.

### Reduktionsstufe II

3,3 kg rohes Rhein-9-anthron-8-glucosid aus Stufe I werden in einer Mischung aus 42 l demineralisiertem Wasser und 7,4 l Butanol-2 suspendiert. Mit 2 l 20%iger (w) Natronlauge und 9,9 kg Trinatriumcitrat wird die Suspension in Lösung gebracht und danach mit 3,3 kg Natriumdithionit und 350 l wassergesättigtem Butanol-2 (z.B. aus Stufe III) versetzt. Der Ansatz wird auf 42 - 45°C aufgeheizt. Der pH-Wert wird mit 20%iger (w) Natronlauge bei 8,5 bis 9 gehalten. Das Reduktionspotential (gegen Ag/AgCl-Elektrode) wird erforderlichenfalls durch Natriumdithionit-Zugabe auf unter -750 mV gehalten.

Nach einer Standzeit von 30 Min. wird die Oberphase entnommen und die Unterphase in der Stufe III weiterverarbeitet.

### Reduktionsstufe III

Mit der Unterphase aus der Stufe II wird unter Zusatz der folgenden Chemikalien das in Stufe II beschriebene Reduktions-/Extraktionsverfahren wiederholt:
1,65 kg Natriumdithionit
0,8 l 20%ige (w) Natronlauge
350 l wassergesättigtes Butanol-2 (z.B. aus Stufe IV)

### Reduktionsstufen IV - VII

Mit der Unterphase aus der jeweils vorausgegangenen Stufe wird unter Zusatz der folgenden Chemikalien das in Stufe II beschriebene Reduktions-/Extraktionsverfahren wiederholt:
0,825 kg Natriumdithionit
0,4 l 20%ige (w) Natronlauge
350 l wassergesättigtes Butanol-2 (z.B. aus den jeweils nachfolgenden Stufen)
Die in der Stufe VII abgetrennte Unterphase wird auf 30 - 35°C gekühlt und das Rhein-9-anthron-8-glucosid wie in Stufe I beschrieben ausgefällt. Der entstandene Niederschlag wird abfiltriert und mit 100 l entmineralisiertem Wasser gewaschen. Anschließend wird mit 10 l Eisen-III-sulfatlösung (Herstellung siehe Stufe B, Beispiel 1) überdeckt.

Das Rhein-9-anthron-8-glucosid wird dann, wie im Beispiel 1 beschrieben, in Diacetylrhein überführt.

### Pharmakologische Untersuchungen

Die Wirksamkeit von Diacetylrhein wurde bei chronischen Entzündungsmodellen nach oraler Verabreichung bestimmt. Folgende Versuchsmodelle kamen zur Anwendung:
Cotton-Pellet-Granulom bei der Ratte und durch intraartikuläre Applikation von Vitamin A ausgelöste Arthrose beim Kaninchen.
a) Cotton-Pellet-Granulom bei der Ratte.
   Junge, geschlechtsreife Ratten (n = 10) erhielten 25, 50 oder 100 mg Diacetylrhein/kg bzw. 5 mg Indomethacin/kg oder 100 mg Acetylsalicylsäure/kg täglich für 5 Tage. Eine nur mit Wasser behandelte Kontrollgruppe wurde ebenfalls mitgeführt. Die Implantation der Pellets erfolgte am ersten Behandlungstag. Frisch- und Trockengewichte der am Versuchsende präparierten Granulome zeigten eine signifikante und deutliche dosisabhängige Verringerung im Vergleich zur Kontrollgruppe. Dabei entsprach die Wirkung von 100 mg Diacetylrhein/kg in etwa der Wirkung von 5 mg Indomethacin oder 100 mg Acetylsalicylsäure. Die Thymus- und Nebennierengewichte änderten sich während der Behandlung nicht.
b) Vitamin-A-Arthrose.
   Durch drei intraartikuläre Injektionen von 30 000 IE Vitamin A während 9 Tagen wurde in zwei Gruppen von je 10 Kaninchen (weiße Neuseeländer) eine arthroseähnliche Gelenkveränderung ausgelöst. 56 Tage später wurden 10 Tiere mit 3 mg Diacetylrhein/kg/Tag für 8 Wochen behandelt. Im Vergleich zur Kontrollgruppe waren die makroskopisch und mikroskopisch erkennbaren Gelenkveränderungen in der Behandlungsgruppe signifikant verringert.
   Die kurative Wirkung von Diacetylrhein wurde weiterhin mit der von Acetylsalicylsäure an je 7 Kaninchen verglichen, die nach 6-tägiger Vorbehandlung mit dreimal 10 000 IE Vitamin A und einem 26-tägigen behandlungsfreien Intervall für 8 Wochen entweder 5 mg Diacetylrhein/kg/Tag (Versuchsgruppe), 15 mg Acetylsalicylsäure/kg/Tag (positive Kontrollgruppe) erhielten oder unbehandelt blieben (negative Kontrollgruppe). In allen drei Gruppen traten 24 Tage nach der letzten Vitamin A-Injektion vergleichbare Bewegungsstörungen in Form von nachziehender Hinterläufe auf. In der negativen Kontrollgruppe verstärkten sich während der folgenden 8 Wochen die klinischen Zeichen einer manifesten Arthrose. In der Versuchsgruppe und der positiven Kontrollgruppe besserten sich diese Symptome unter der 8-wöchigen Behandlung signifikant.

### Magenschleimhautveränderungen

Während die einmalige Gabe von 400 mg Diacetylrhein/kg oder des Lösungsmittels bei der Ratte keine Erosionen der Magenschleimhaut verursachte, fanden sich nach Gabe von Ibuprofen (200 mg/kg) oder Indomethacin (20 mg/kg) eindeutige Schleimhautschäden von punktförmigen (1 mm Durchmesser) bis großen (3 mm Durchmesser) Erosionen. Auch die zweimal tägliche Gabe von 100 mg Diacetylrhein/kg über 3 Tage löste keine Schleimhautschäden aus, wohl aber die entsprechende Anwendung von 10 mg Indomethacin/kg. Dabei handelte es sich um Erosionen von 1 - 3 mm Durchmesser.

### Toxikologie

Die akute Toxizität LD₅₀ beträgt je nach untersuchter Spezies (Ratte, Maus, Katze) nach oraler Anwendung 1,9 bis 7,9 g/kg. Dabei erwies sich die Ratte als am wenigsten empfindlich. Nach parenteraler Gabe (i.v.;i.p.) lagen die LD₅₀-Werte bei diesen Spezies zwischen 119 und 339 mg/kg.

### Klinische Untersuchungen

1. In einer Doppelblind-Studie gegen Naproxen und anschließender Plazebo-Nachbehandlung wurde die Wirkung von Diacetylrhein bei Coxarthrose und Gonarthrose bei 95 (49/46) Patienten untersucht. Die applizierte Dosis war 50 mg Diazetylrhein 2 mal täglich bzw. 750 mg Naproxen täglich. Die Behandlungsdauer war 60 Tage nach 7-tägiger wash-out-Phase. Die anschließende Plazebo-Behandlung erstreckte sich über 60 Tage.
   Prüfgrößen waren die Schmerz- und Beweglichkeits-Symptomatik nach einer Score-Scala, Funktionseinschränkung und Verträglichkeit.
   In beiden Behandlungsgruppen (Diacetylrhein/Naproxen) wurde hinsichtlich aller Prüfparameter eine statistisch signifi-signifikante Besserungsrate (P < 0,01 bzw. P < 0,05) im Vergleich zu den Ausgangswerten festgestellt. Nach Absetzen der Behandlung und anschließender Plazebogabe zeigte sich jedoch für die Diacetylrhein/Plazebo-Gruppe an den Tagen 90 und 120 hinsichtlich der Parameter Spontanschmerz, aktiver und passiver Bewegungsschmerz, eine statistisch signifikante Überlegenheit (P < 0,01) im Vergleich zum Naproxen-/Plazebo-Kollektiv. Dieser Unterschied wurde auf dem 5 %-Niveau auch für die Variablen Nachtschmerz und Druckschmerz 30 Tage nach Absetzen von Diacetylrhein gesichert.
2. In einer offenen Verlaufsstudie mit Kontrolle wurde die Wirkung von Diacetylrhein gegen Osteoarthrose der Wirbelsäule und des Knies bei 70 Patienten (35/35) untersucht. Die applizierte Dosis war 100 mg Diacetylrhein pro Tag. Die Behandlungsdauer betrug 60 Tage, die Beobachtungsdauer 75 Tage. Die Prüfgrößen waren Schmerz- und Bewegungseinschränkung. Die Größen wurden nach einem Score-System ermittelt.
   Die Kontrollgruppe umfaßte 35 Patienten, bei denen ausschließlich physio-therapeutische Maßnahmen durchgeführt wurden. In der Diacetylrhein-Behandlungsgruppe wurde ebenfalls eine Physiotherapie durchgeführt.
   Die Auswertung der Ergebnisse zeigte bezüglich aller Parameter eine statistisch signifikante Überlegenheit der Behandlungsgruppe gegenüber der Kontrollgruppe. Auch nach Absetzen der Behandlung konnte für die Diacetylrhein-Gruppe ein anhaltender therapeutischer Effekt ("hang-over-Effekt) festgestellt werden.
3. In einer single-blind, cross-over-Studie gegen Naproxen wurde die Wirkung von Diacetylrhein bei lokalisierter Arthrose bei 20 Patienten untersucht. Diese wurden in zwei Gruppen eingeteilt, wobei in der ersten Gruppe zunächst 2 mal 50 mg Diacetylrhein 20 Tage lang verabreicht wurden. Anschließend erfolgte drei Tage lang eine wash-out-Phase und eine weitere Behandlung mit 2 mal 250 mg Naproxen pro Tag an weiteren 20 Tagen. In der zweiten Gruppe wurde die umgekehrte Reihenfolge eingehalten. Die Behandlungsdauer betrug insgesamt 43 Tage. Prüfgrößen waren Schmerz, Kompressionsschmerz, passiver Bewegungsschmerz, Funktionseinschränkung und Schwellung nach einem Score-System.
   Die Auswertung der Ergebnisse zeigt eine Überlegenheit der Behandlung mit Diacetylrhein im Vergleich zur Behandlung mit Naproxen. Es wurden keine nennenswerten Nebenwirkungen beobachtet, auch keine Veränderungen der klinischen Laborparameter.
4. In einer randomisierten Doppelblindstudie in "double dummy-Technik" gegen Naproxen wurde die Wirkung von Diacetylrhein bei 23 Patienten (12/11) mit Osteoarthrose untersucht (Verträglichkeitsstudie). Die applizierte Dosis war 2 mal 50 mg Diacetylrhein pro Tag und 3 mal 250 mg Naproxen pro Tag. Die Behandlungsdauer betrug 4 Wochen. Die Prüfgrößen waren ösophagogastroduodenoskopische Befunderhebung vor und nach Therapie. Es wurden nur Patienten mit normalen Schleimhautbefunden bzw. mit leichten Schleimhautläsionen (Grad 1) in die Studie aufgenommen.
   Nach 4 Wochen zeigten die endoskopischen Befunde in einem Fall (10 %) in der Diacetylrheingruppe Schleimhautläsionen des Grades 2, während in der Naproxen-Behandlungsgruppe 5 Patienten (50 %) Schleimhautläsionen der Grade 2, 3 und 4 aufwiesen. In allen Fällen lag ein normaler Aufnahmebefund vor.

### Analytische Bestimmung von Aloeemodin

Man löst 50 mg Diacetylrhein in 25,3 ml 0,5 M NaOH in einem Scheidetrichter und schüttelt 10 Minuten. Anschließend gibt man 74,6 ml einer Lösung zu, die 0,5 M Glycin Und 0,5 M NaCl enthält. Dabei ergibt sich ein pH von 9,5.

Diese Lösung extrahiert man 3 mal mit 25 ml Chloroform. Die vereinigten organischen Phasen werden 1 mal mit 10 ml 0,5 M eines Puffers vom pH 9,5 (Glycin, NaOH, NaCl) und 1 mal mit 10 ml 0,01 M Schwefelsäure extrahiert. Man entfernt das Lösungsmittel der organischen Phase und löst den Rückstand in 1 ml Methanol.

Für die Standardlösung löst man 2 mg Aloeemodin in 20 ml N, N-Dimethylacetamid und verdünnt mit Methanol bis zu einer Konzentration von 2 µg/ml (entsprechend 40 ppm).

Der Gehalt der Lösungen wird mittels HPLC untersucht. Die Linearität der HPLC-Methode wurde mit Aloeemodin-Standardlösung im Bereich von 0,11 µg/ml (entsprechend 2,2 ppm) bis 53,6 µg/ml (entsprechend 1072 ppm) nachgewiesen. Die Gehaltsbestimmung erfolgt mit einer Merck HPLC-Säule Lichrocart 250-4, gepackt mit LiChrospher-100 RP-18, 5 µm, bei 40 °C mit einer mobilen Phase aus 1 %iger Essigsäure in Methanol (v/v), 1 %iger Essigsäure in Wasser (v/v) und Acetonitril im Verhältnis von 49:46:5.

## Patentansprüche

1. Verfahren zur Gewinnung von Diacetylrhein,
**dadurch gekennzeichnet,** daß man Aloeemodinkomponente enthaltendes Diacetylrhein einer Flüssig-Flüssig-Verteilung zwischen einem nur teilweise mit Wasser mischbaren, polaren organischen Lösungsmittel und einer wäßrigen Phase von pH 6,5 bis 7,5 unterwirft und das Diacetylrhein gewinnt und gegebenenfalls umkristallisiert.

2. Verfahren nach Anspruch 1
**dadurch gekennzeichnet,** daß man
A) Aloeemodinkomponenten enthaltendes Rheinanthron-8-glucosid zu den entsprechenden Anthrachinonverbindungen oxidiert,
B) den Glucoserest in 8-Stellung der Azithrachinonverbindungen in saurem Medium abspaltet,
C) die erhaltenen 1,8-Dihydroxyanthrachinonverbindungen enthaltend Aloeemodinkomponenten acetyliert und
D) eine Flüssig-Flüssig-Verteilung des erhaltenen Produktes zwischen einem nur teilweise mit Wasser mischbaren, polaren organischen Lösungsmittel und einer wäßrigen Phase vom pH 6,5 bis 7,5 vornimmt und das Diacetylrhein gewinnt und gegebenenfalls umkristallisiert.

3. Verfahren nach Anspruch 1
**dadurch gekennzeichnet,** daß man
A) ein Sennosidgemisch einer Reduktion zu den entsprechenden Anthronverbindungen unterwirft,
B) die erhaltenen Anthronverbindungen zu den entsprechenden Anthrachinonverbindungen oxidiert,
C) den Glucoserest in 8-Stellung der Anthrachinonverbindungen in saurem Medium abspaltet,
D) die erhaltenen 1,8-Dihydroxyanthrachinonverbindungen enthaltend Aloeemodlnkomponenten acetyliert und
E) eine Flüssig-Flüssig-Verteilung des erhaltenen Produktes zwischen einem nur teilweise mit Wasser mischbaren, polaren organischen Lösungsmittel und einer wäßrigen Phase vom pH 6,5 bis 7,5 vornimmt und das Diacetylrhein gewinnt und gegebenenfalls umkristallisiert.

4. Verfahren nach einem der vorhergehenden Ansprüche,
**dadurch gekennzeichnet,** daß man für die Flüssig-Flüssig-Verteilung als polares organisches Lösungsmittel 2-Butanon verwendet.

5. Verfahren nach einem der vorhergehenden Ansprüche,
**dadurch gekennzeichnet,** daß man für die Flüssig-Flüssig-Verteilung eine acetatgepufferte wäßrige Phase verwendet.

6. Verfahren nach einem der vorhergehenden Ansprüche,
**dadurch gekennzeichnet,** daß man die Flüssig-Flüssig-Verteilung im Gegenstromverfahren durchführt.

7. Verfahren nach einem der Ansprüche 2 bis 6,
**dadurch gekennzeichnet,** daß man als Oxidationsmittel ein Eisen-III-Salz, vorzugsweise Eisen-III-Sulfat, verwendet.

8. Verfahren nach Anspruch 3,
**dadurch gekennzeichnet,** daß das Sennosid-Gemisch erhältlich ist durch Extraktion der Sennadroge mit wäßrigem Methanol, vorzugsweise in Gegenwart eines Puffers.

9. Verfahren nach einem der Ansprüche 3 bis 8,
**dadurch gekennzeichnet,** daß man in Stufe A) als Reduktionsmittel ein Alkalimetalldithionit verwendet.

10. Verfahren nach Anspruch 9,
**dadurch gekennzeichnet,** daß man bei einem pH-Wert von 7 bis 9 arbeitet.

11. Verfahren nach einem der Ansprüche 3 bis 10,
**dadurch gekennzeichnet,** daß man die Reduktion mehrfach durchführt.

12. Verfahren nach einem der vorhergehenden Ansprüche,
**dadurch gekennzeichnet,** daß man das erhaltene Diacetylrhein umkristallisiert, indem man das Diacetylrhein in ein Alkalimetallsalz überführt, dieses in einem wäßrigen C₁-C₃-Alkohol aufnimmt und das Diacetylrhein durch Zugabe einer Säure wieder ausfällt.

13. Verfahren nach einem der Ansprüche 1 bis 11,
**dadurch gekennzeichnet,** daß man das erhaltene Diacetylrhein aus Ethyllactat umkristallisiert.

14. Pharmazeutisches Mittel, enthaltend Diacetylrhein mit weniger als 20 ppm Aloeemodinkomponenten zusammen mit üblichen pharmazeutischen Trägern und Hilfsstoffen.

## Claims

1. Method for obtaining diacetylrhein, characterised in that diacetylrhein containing aloe-emodin components is subjected to a liquid-liquid partition between a polar organic solvent which is only partly miscible with water and an aqueous phase of pH 6.5 to 7.5 and the diacetylrhein is recovered and optionally recrystallised.

2. Method according to claim 1, characterised in that
A) rhein-9-anthron-8-glucoside containing aloe-emodin components is oxidised to the corresponding anthraquinone compounds,
B) the glucose radical in the 8-position of the anthraquinone compounds is split off in an acid medium,
C) the 1,8-dihydroxyanthraquinone compounds obtained containing aloe-emodin components are acetylated and
D) a liquid-liquid partition of the product obtained is carried out between a polar organic solvent which is only partly miscible with water and an aqueous phase of pH 6.5 to 7.5 and the diacetylrhein is recovered and optionally recrystallised.

3. Method according to claim 1, characterised in that
A) a mixture of sennosides is reduced to the corresponding anthrone compounds,
B) the anthrone compounds obtained are oxidised to the corresponding anthraquinone compounds,
C) the glucose radical in the 8-position of the anthraquinone compounds is split off in an acid medium,
D) the 1,8-dihydroxyanthraquinone compounds obtained containing aloe-emodin components are acetylated and
E) a liquid-liquid partition of the product obtained is carried out between a polar organic solvent which is only partly miscible with water and an aqueous phase of pH 6.5 to 7.5 and the diacetylrhein is recovered and optionally recrystallised.

4. Method according to any one of the preceding claims, characterised in that 2-butanone is used as polar organic solvent for the liquid-liquid partition.

5. Method according to any one of the preceding claims, characterised in that an acetate-buffered aqueous phase is used for the liquid-liquid partition.

6. Method according to any one of the preceding claims, characterised in that the liquid-liquid partition is carried out in a countercurrent process.

7. Method according to any one of claims 2 to 6, characterised in that an iron(III) salt, preferably iron(III) sulphate, is used as oxidising agent.

8. Method according to claim 3, characterised in that the mixture of sennosides is obtainable by extraction of the senna drug with aqueous methanol, preferably in the presence of a buffer.

9. Method according to any one of claims 3 to 8, characterised in that an alkali metal dithionite is used as reducing agent in Step A.

10. Method according to claim 9, characterised in that the procedure is carried out at a pH value of from 7 to 9.

11. Method according to any one of claims 3 to 10, characterised in that the reduction is carried out several times.

12. Method according to any one of the preceding claims, characterised in that the diacetylrhein obtained is recrystallised by converting the said diacetylrhein into an alkali metal salt, by taking this up in an aqueous C₁ - C₃ alcohol and again precipitating out the diacetylrhein through the addition of an acid.

13. Method according to any one of claims 1 to 11, characterised in that the diacetylrhein obtained is recrystallised from ethyl lactate.

14. Pharmaceutical preparation containing diacetylrhein having less than 20 ppm of aloe-emodin components together with conventional pharmaceutical carriers and auxiliary substances.

## Revendications

1. Procédé de préparation de diacétylrhéine, caractérisé en ce que l'on soumet une diacétylrhéine contenant des constituants de type aloémodine à un partage liquide-liquide entre un solvant organique polaire qui n'est que partiellement miscible à l'eau et une phase aqueuse de pH 6,5 à 7,5, et on obtient la diacétylrhéine que l'on recristallise éventuellement.

2. Procédé selon la revendication 1, caractérisé en ce que
A) on oxyde un rhéine-anthrone-8-glucoside contenant des constituants de type aloémodine pour former les composés anthraquinoniques correspondants,
B) on élimine en milieu acide le reste de glucose en position 8 des composés anthraquinoniques,
C) on acétyle les composés 1,8-dihydroxyanthraquinoniques obtenus contenant des constituants de type aloémodine et
D) on effectue un partage liquide-liquide du produit obtenu entre un solvant organique polaire qui n'est que partiellement miscible à l'eau et une phase aqueuse de pH 6,5 à 7,5, et on obtient la diacétylrhéine que l'on recristallise éventuellement.

3. Procédé selon la revendication 1, caractérisé en ce que
A) on soumet un mélange de sennosides à une réduction pour former les composés d'anthrone correspondants,
B) on oxyde les composés d'anthrone obtenus pour former les composés anthraquinoniques correspondants,
C) on élimine en milieu acide le reste de glucose en position 8 des composés anthraquinoniques,
D) on acétyle les composés 1,8-dihydroxyanthraquinoniques obtenus contenant des constituants de type aloémodine et
E) on effectue un partage liquide-liquide du produit obtenu entre un solvant organique polaire qui n'est que partiellement miscible à l'eau et une phase aqueuse de pH 6,5 à 7,5, et on obtient la diacétylrhéine que l'on recristallise éventuellement.

4. Procédé selon l'une des revendications précédentes, caractérisé en ce que l'on utilise de la 2-butanone comme solvant organique polaire pour le partage liquide-liquide.

5. Procédé selon l'une des revendications précédentes, caractérisé en ce que l'on utilise une phase aqueuse tamponnée à l'acétate pour le partage liquide-liquide.

6. Procédé selon l'une des revendications précédentes, caractérisé en ce que l'on effectue le partage liquide-liquide à contre-courant.

7. Procédé selon l'une des revendications 2 à 6, caractérisé en ce que l'on utilise comme agent d'oxydation un sel de fer III, de préférence le sulfate de fer III.

8. Procédé selon la revendication 3, caractérisé en ce que l'on peut obtenir le mélange de sennosides par extraction de la drogue de séné avec du méthanol aqueux, de préférence en présence d'un tampon.

9. Procédé selon l'une des revendications 3 à 8, caractérisé en ce que, dans l'étape A), on utilise un dithionite de métal alcalin comme agent réducteur.

10. Procédé selon la revendication 9, caractérisé en ce que l'on travaille à un pH compris entre 7 et 9.

11. Procédé selon l'une des revendications 3 à 10, caractérisé en ce que l'on effectue plusieurs fois la réduction.

12. Procédé selon l'une des revendications précédentes, caractérisé en ce que l'on recristallise la diacétylrhéine obtenue en transformant la diacétylrhéine en un sel de métal alcalin que l'on reprend dans un alcool en C₁-C₃ aqueux et en reprécipitant la diacétylrhéine par addition d'un acide.

13. Procédé selon l'une des revendications 1 à 11, caractérisé en ce que l'on recristallise la diacétylrhéine obtenue dans du lactate d'éthyle.

14. Produit pharmaceutique contenant de la diacétylrhéine qui comporte moins de 20 ppm de constituants de type aloémodine, avec des supports et produits auxiliaires pharmaceutiques classiques.
